# EUROPEAN PATENT APPLICATION

(11) **EP 1 535 572 A1**
(43) Date of publication of application: **01.06.2005**
(21) Application number: 03794129.1
(22) Date of filing: 29.08.2003
(51) Int. Cl.: A61B 5/15

(54) **INVASIVE APPLIANCE**

(30) Priority: 05.09.2002 JP 2002260445
(71) Applicant: MATSUSHITA ELECTRIC INDUSTRIAL CO., LTD., Kadoma-shi, Osaka 571-8501 (JP)
(72) Inventor: NAKAYAMA, Hiroshi, Hirakata-shi, Osaka 573-1114 (JP); EMOTO, Fumiaki, Hirakata-shi, Osaka 573-0051 (JP); HIRATSUKA, Atsunori, Sagamihara-shi, Kanagawa 229-1103 (JP)
(74) Representative: Grünecker, Kinkeldey, Stockmair & Schwanhäusser Anwaltssozietät
(86) International application number: PCT/JP2003/011006
(87) International publication number: WO 2004/021886

(57) **Abstract**

An injector or lancet inducing much less pain is provided.

An invasive apparatus of obtaining an in vivo fluid,
wherein the invasive apparatus includes a different stimulus applying portion 1a of applying a stimulus different from a stimulus applied by an invasive portion 3 to an organism in a leading end area other than a hole 7 through which the invasive portion 3 exits,
the invasive portion 3 is a hollow tube, knife, needle or laser beam,
the different stimulus is a mechanical stimulus,
a recessed portion and/or raised portion is formed in the area other than the hole 7,
the different stimulus applying portion 1a is the recessed portion and/or raised portion, and
the different stimulus is given by the recessed portion and/or raised portion.

## Description

### Technical Field

The present invention relates to an invasive apparatus of collecting a body fluid such as blood being a measurement object, an invasive method and an in vivo fluid measuring apparatus.

### Background Art

Numerous means of alleviating a pain when invading the surface of an organism have been practiced.

For example, Japanese Patent Laid-Open No. 07-51251 describes alleviation of a pain using an apparatus comprised of a decompression chamber, a suction chamber having puncturing means, and puncture releasing means.

Furthermore, for example, Japanese Patent Laid-Open No. 07-255706 describes alleviation of a pain by a simple blood collection apparatus comprised of decompressing means, an airtight suction chamber having puncturing means, and puncture releasing means of indirectly release-driving puncture of the puncturing means.

Furthermore, for example, Japanese Patent Laid-Open No. 08-187237 provides a blood collection needle with which blood in an amount required for diagnosis is made to effuse on the skin without causing a pain by processing the blood collection needle.

Furthermore, for example, Japanese Patent Laid-Open No. 08-317917 describes alleviation of a pain with respect to an apparatus comprised of suctioning means of suctioningthe skin under decompression, puncturingmeans of making a puncturing member bump into the skin in the suctioned state, and releasing means of pulling out the bumping puncturing member from the skin.

Furthermore, for example, Japanese Patent Laid-Open No. 08-317918 provides a blood collection apparatus having puncturing means comprised of a multi-needle structure.

Furthermore, for example, Japanese Patent Laid-Open No. 08-837148 describes alleviation of a pain with respect to a blood collection apparatus comprising suctioning means of suctioning the skin under decompression, puncturing means of making a puncturing member bump into the skin in the suctioned state, and releasing means of separating the bumping puncturing member from the skin, and a blood collection apparatus having puncturing means comprised of a multi-needle structure.

Furthermore, for example, Japanese Patent Laid-Open No. 09-804707 describes alleviation of a pain with respect to provision of an assembly attachable to an injector capable of adjusting the piercing depth of a piercing element more easily.

Furthermore, for example, Japanese Patent Laid-Open No. 10-127610 describes alleviation of a pain with respect to a blood sample collection apparatus having means of creating a vacuum in a housing when a lancet shifts from its contraction state to its extension state.

Furthermore, for example, National Publication of International Patent Application No. 2001-515377 describes an apparatus having diagnostic tests combined with a sensor of collecting a blood sample, wherein a differential gas pressure is used for pushing a lancet into a skin tissue when the blood sample is collected. The document describes alleviation of a pain in this way.

Furthermore, for example, Japanese Patent Laid-Open No. 11-164825 describes alleviation of a pain with respect to a method in which a ring is placed against the skin and an elastic pressure is repeatedly applied to the ring, whereby a body fluid is collected through breaks in the skin.

Furthermore, for example, Japanese Patent Laid-Open No. 11-76211 describes a blood sample collection apparatus incorporating a lancet to pierce the skin, comprising lancet attenuating means of preventing a situation in which a lancet holder vibrates to give an unnecessary pain after the skin is stung.

Furthermore, for example, National Publication of International Patent Application No. 2001-524343 describes a method and apparatus of obtaining an interstitial fluid from a patient for diagnostic tests such as glucose monitoring, comprising a step of using a vacuum and skin extension to draw out the interstitial fluid through openings of the skin. The document describes alleviation of a pain as an effect thereof.

Furthermore, for example, in Japanese Patent Laid-OpenNo.2000-237172,apiercingapparatuscomprises a lancet holder under a load with a spring slidably mounted in a housing to support a disposable lancet and needle. A knob in the rear part of the apparatus has a forward-extending finger stopping the lancet holder at a predetermined point that can be adjusted after the apparatus is launched. The document describes that the finger not only controls the penetration depth of the needle, but also absorbs vibrations, and thereby alleviates a pain of a user.

Furthermore, for example, Japanese Patent Laid-Open No. 2002-696 describes a needle for acupuncture. A needle body in the needle for acupuncture comprises a body portion having almost a uniform sectional form along the length, and a needle tip portion continuously extending from the body portion and tapering in a bullet form. Where the section diameter D of the body portion is 1, the curvature radius of an outer edge curve extending along the long axis from a tip A of the needle tip portion to a position of 0.6 is in the range of 1.3 to 3. The document describes alleviation of a pain of a patient during penetration in this way.

Furthermore, for example, Japanese Patent Laid-Open No. 2001-161815 describes an injector. The leading end of a cylindrical portion attached to the injector stimulates the skin, whereby the site inside the stimulated skin loses a sense. The document describes alleviation of a pain of a patient during injection in this way.

Furthermore, the entire disclosure of Japanese Patent Laid-Open No. 07-51251, Japanese Patent Laid-Open No. 07-255706, Japanese Patent Laid-Open No. 08-187237, Japanese Patent Laid-Open No. 08-317917, Japanese Patent Laid-Open No. 08-317918, Japanese Patent Laid-Open No. 08-837148, Japanese Patent Laid-Open No. 09-804707, Japanese Patent Laid-Open No. 10-127610, National Publication of International Patent Application No. 2001-515377, Japanese Patent Laid-Open No. 11-164825, Japanese Patent Laid-Open No. 11-76211, National Publication of International Patent Application No. 2001-524343, Japanese Patent Laid-Open No. 2000-237172, Japanese Patent Laid-Open No. 2002-696 and Japanese Patent Laid-Open No. 2001-161815 are incorporated herein by reference in its entirety.

There is a disadvantage that a considerable pain remains in the method of alleviation of a pain by reducing the invasion depth where possible, sharpening the shape of a needle, reducing the thickness, or providing vibration preventingmeasures to avoid repeated puncture, which is one of the conventional methods.

That is, the conventional method has a problem such that a considerable pain remains.

Furthermore, in the method by a suction system, a speed of evaporation of water in a body fluid is significantly enhanced due to suction during collection of the body fluid, and thus the concentration of a measurement obj ect in the body fluid increases, or a speed of coagulation of blood is enhanced. As a result, significant errors are caused in measurement values.

That is, the method by a suction system has a problem such that the concentration of a measurement object in a body fluid increases, or a speed of coagulation of blood is enhanced, resulting in significant errors in measurement values.

Furthermore, stimulating means is provided at the leading end of a fixture of an injector, and the means is used to provide alleviation of a pain during injection, but there is a problem such that in the case of injection, a drug is injected, or a body fluid is collected in a syringe and therefore, during this operation, the pain cannot be alleviated by the stimulating means.

### Disclosure of the Invention

An object of the present invention is to provide an invasive apparatus causing less pain, an invasive method, and an in vivo fluid measuring apparatus in consideration of the above problems.

Furthermore, an object of the present invention is to provide an in vivo fluid measuring apparatus that can measure an object in a body fluid more accurately.

In order to solve the above problems, the first invention is an invasive apparatus of obtaining an in vivo fluid, comprising a different stimulus applying portion of applying a stimulus different from a stimulus applied by an invasive portion to an organism in a leading end area other than a hole through which said invasive portion goes out.

Furthermore, the second invention is the invasive apparatus of the first invention, wherein the in vivo fluid is obtained from the depth of 3 mm or less under the skin.

Furthermore, the third invention is the invasive apparatus of the first invention, wherein the different stimulus applying portion is detachable.

Furthermore, the fourth invention is the invasive apparatus of the first invention, wherein the invasive portion is a hollow tube, knife, needle or laser beam.

Furthermore, the fifth invention is the invasive apparatus of the first invention, wherein the different stimulus is given continuously or intermittently.

Furthermore, the sixth invention is the invasive apparatus according to the first invention, wherein said different stimulus is a mechanical stimulus, electric stimulus, optical stimulus or change in temperature.

Furthermore, the seventh invention is the invasive apparatus according to the sixth invention, wherein said different stimulus is a mechanical stimulus,
a recessed portion and/or a raised portion is formed in the area other than said hole,
said different stimulus applying portion is said recessed portion and/or raised portion, and
said different stimulus is given by the recessed portion and/or raised portion.

Furthermore, the eighth invention is the invasive apparatus of the first invention, wherein the different stimulus is given before the invasive portion contacts the surface of the organism.

Furthermore, the ninth invention is the invasive apparatus according to the seventh invention, wherein said recessed portion and/or raised portion is at a distance of from at least 1 mm to 20 mm from an area in which said invasive portion contacts the surface of the organism.

Furthermore, the tenth invention is the invasive apparatus according to the ninth invention, wherein said surface of the organism is a finger tip, and
said recessed portion and/or raised portion is at a distance of from at least 1 mm to 10 mm from an area in which said invasive portion contacts said finger tip.

Furthermore, the eleventh invention is the invasive apparatus according to the tenth invention, wherein the invasive apparatus is a lancet apparatus of collecting blood.

Furthermore, the twelfth invention is the invasive apparatus of the seventh invention, wherein the raised portion has a protrusive portion of 0.1 mm or greater.

Furthermore, the thirteenth invention is the invasive apparatus of the seventh invention, wherein the angle at which the raised portion contacts the surface of the organism is 5 degrees or greater.

Furthermore, the fourteenth invention is the invasive apparatus of the seventh invention, wherein the recessed portion and/or raised portion are situated continuously or discontinuously on the outer periphery of the area in which the invasive portion contacts the surface of the organism.

Furthermore, the fifteenth invention is the invasive apparatus of the seventh invention, wherein the recessed portion and/or raised portion are constituted by a rolling body rolling on the surface of the organism.

Furthermore, the sixteenth invention is the invasive apparatus of the fifteenth invention, wherein the center of gravity of the rolling body is decentered with respect to the center of rotation of the rolling body.

Furthermore, the seventeenth invention is the invasive apparatus of the seventh invention, wherein the recessed portion and/or raised portion are constituted by a vibration body giving vibrations on the surface of the organism.

Furthermore, the eighteenth invention is the invasive apparatus of the seventeenth invention, wherein the vibration body produces vibrations by a magnetic coil or piezoelectric element.

Furthermore, the nineteenth invention is the invasive apparatus of the seventeenth invention, wherein the amplitude and/or the frequency of the vibration body are variable.

Furthermore, the twentieth invention is the invasive apparatus of the seventh invention, wherein a pressure applied to the surface of the organism by the recessed portion and/or raised portion is at least 1 kgf/cm².

Furthermore, the twenty-first invention is the invasive apparatus of the seventh invention, wherein the load application speed of a mechanical stimulus given to the surface of the organism by the recessed portion and/or raised portion is 0.1 Kgf/sec·cm².

Furthermore, the twenty-second invention is the invasive apparatus of the seventh invention, wherein a time point 0.1 to 5 seconds before invasion by the invasive portion is a point of maximum load application of a mechanical stimulus given to the surface of the organism by the recessed portion and/or raised portion.

Furthermore, the twenty-third invention is the invasive apparatus of the seventh invention, the recessed portion and/or raised portion being constituted by at least a first raised portion and a second raised portion,
wherein the different stimulus is given by sandwiching the surface of the organism between the first raised portion and the second raised portion.

Furthermore, the twenty-fourth invention is the invasive apparatus of the first invention, further comprising a sealed air body,
wherein air is encapsulated in the sealed air body, whereby the surface of the organism is pressed against the different stimulus applying portion of the leading end portion.

Furthermore, the twenty-fifth invention is the invasive apparatus of the first invention, comprising:
organism insertion guiding means of inserting the organism; and
organism holding means of holding the organism,
wherein the organism holding means holds down the organism from the side opposite to the area in which the invasive portion contacts the organism, whereby the organism is pressed against the different stimulus applying portion.

Furthermore, the twenty-sixth invention is an in vivo fluid measuring apparatus comprising:
the invasive apparatus of the first invention;
fluid transporting means of transporting an in vivo fluid obtained by invasion by the invasive apparatus; and
a measurement unit,
wherein the measurement unit analyzes the collected in vivo fluid.

Furthermore, the twenty-seventh invention is an invasive method of obtaining an in vivo fluid,
wherein a stimulus different from a stimulus by invasion means is given to an area near the position of the invasion.

### Brief Description of the Drawings

Figure 1 (a) is a perspective view of an invasive apparatus in the first embodiment of the present invention;
Figure 1(b) is a perspective view of the invasive apparatus in the first embodiment of the present invention;
Figure 1(c) is a perspective view of the invasive apparatus in the first embodiment of the present invention;
Figure 2 is a perspective view of the invasive apparatus in the second embodiment of the present invention;
Figure 3 is a perspective view of the invasive apparatus in the third embodiment of the present invention;
Figure 4 is a sectional view of the invasive apparatus in the fourth embodiment of the present invention;
Figure 5 is a sectional view of the invasive apparatus in the fifth embodiment of the present invention;
Figure 6 is a sectional view of the invasive apparatus in the sixth embodiment of the present invention;
Figure 7 is a sectional view of the invasive apparatus in the seventh embodiment of the present invention;
Figure 8(a) is a perspective view of the invasive apparatus with a detachable stimulus applying portion in the first embodiment of the present invention;
Figure 8(b) is a perspective view of the invasive apparatus with a detachable stimulus applying portion in the first embodiment of the present invention; and
Figure 8(c) is a perspective view of the invasive apparatus with a detachable stimulus applying portion in the first embodiment of the present invention.

### (Description of the symbols)

- 1a, 1b, 1c: stimulus applying portion
- 2: spring
- 3: lancet
- 4: holder
- 5: spring
- 7: lancet passage port
- 8: vibration body
- 9a, 9b: rolling body
- 10a, 10b, 10c, 10d, 10e, 10f, 10g, 10h, 10i: invasive apparatus
- 11a, 11b: gear
- 12a, 12b: gear
- 16: sealed air body
- 18: finger insertion guiding means
- 21: finger holding means

### Best Mode for Carrying Out the Invention

Embodiments of the present invention will be described below with reference to the drawings.

### (First Embodiment)

First, the first embodiment will be described.

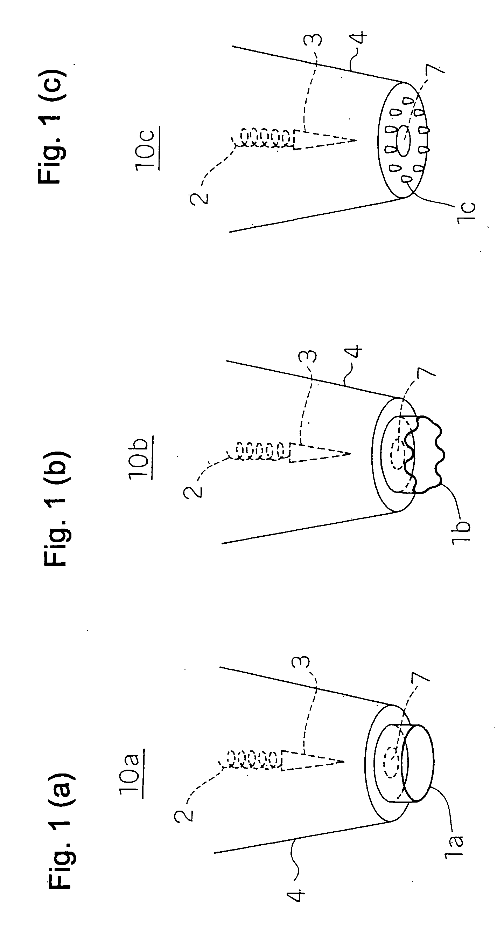

An invasive apparatus 10a of the first embodiment is shown in Figure 1(a). The invasive apparatus 10a is a lancet apparatus of collecting blood, for example.

The invasive apparatus 10a is comprised of a stimulus applying portion 1a, a spring 2, a lancet 3, a holder 4 and a lancet passage port 7.

The stimulus applying portion 1a is means of giving to the surface of an organism a stimulus different from a stimulus given to the surface of the organism when the lancet 3 invades the surface of the organism, and the stimulus applying portion 1a is made of material such as rubber, plastic or metal, and has a certain strength.

The spring 2 is means of protruding the lancet 4 from the lancet passage port 7 for the lancet 4 to invade the surface of the organism.

The lancet 3 is a needle, which is means of puncturing the surface of the organism to collect blood from the surface of the organism.

The holder 4 is an enclosure storing the lancet 3.

The lancet passage port 7 is a hole through which the lancet 3 passes when the surface of the organism is punctured by the lancet 3 to make an invasion.

Specifically, the lancet passage port 7 is provided in the lower part of the holder 4, and the stimulus applying portion 1a is provided in an outer periphery portion at a predetermined distance from the center of the lancet passage port 7. Furthermore, the lancet 3 is stored in the holder 4, and is capable of being protruded from the lancet passage port 7 by the spring 2.

Furthermore, the lancet 3 of this embodiment is an example of an invasive portion of the present invention, the stimulus applying portion of this embodiment is an example of a different stimulus applying portion, and the stimulus applying portion of this embodiment is an example of a recessed portion or raised portion of the present invention.

The operation of this embodiment will now be described.

For collecting blood from the surface of the organism by the invasive apparatus 10a, the invasive apparatus 10a is first brought close to an area of blood collection on the surface of the organism.

Then, the stimulus applying portion 1a of the invasive apparatus 10a is pressed against the periphery of the area of blood collection on the surface of the organism. Consequently, the stimulus applying portion 1a gives a mechanical stimulus to the surface of the organism.

After the expiration of a predetermined time interval from a time when the stimulus applying portion 1a gives a mechanical stimulus to the surface of the organism, the lancet 3 is protruded from the lancet passage port 7 by a pressing pressure of the spring 2 to puncture the surface of the organism to a depth of 3 mm or less to make an invasion, and blood is effused from the surface of the organism. Then, the effused blood is collected.

In this way, the lancet 3 punctures the surface of the organism to a depth of 3 mm or less, whereby a pain during puncture can be alleviated compared to the case where the lancet 3 punctuates the surface of the organism to a depth of 3 mm or greater.

Furthermore, in embodiments other than the first embodiment, the lancet is protruded from the lancet passage port to puncture the surface of the organism to a depth of 3 mm or less, whereby a pain during puncture can be alleviated as a matter of course.

In this way, in this embodiment, a stimulus different from that of the lancet 3 is given by the stimulus applying portion 1a from a time when or before the lancet 3 contacts the surface of the organism. This enables a painful stimulus to be masked in an early stage. Furthermore, unlike the method by a suction process , the concentration of a measurement object in blood never increases, and therefore no significant errors are caused in measurement values of collected blood.

Furthermore, for the injector disclosed in Japanese Patent Laid-Open No. 2001-161815, the leading end of a cylindrical portion attached to the injector stimulates the skin, but the leading end of the cylindrical portion is supported by the spring. Thus, as the leading end of the cylindrical portion is pressed against the skin, the leading end of the cylindrical portion slid and the spring supporting the leading end of the cylindrical portion shrinks, and therefore the stimulus given to the skin by the leading end of the cylindrical portion increases. As an injection needle of the injector invades the skin, the leading end of the cylindrical portion is further slid continuously, and therefore the stimulus given to the skin by the leading end of the cylindrical portion continuously increases. Thus, it is difficult to keep the magnitude of the stimulus given to the skin by the leading end of the cylindrical portion at the optimum while the injection needle of the injector invades the skin. Furthermore, it is difficult for the leading end of the cylindrical portion to give an optimum amount of stimulus to the skin in optimum timing either automatically or manually in synchronization with timing in which the injection needle of the injector invades the skin. Furthermore, the time period over which the injection needle of the injector invades the skin is much longer than the time period over which the lancet 3 of this embodiment invades the skin because an injection solution should be injected during the time period.

In contrast, for the lancet 3 of this embodiment, since the stimulus applying portion 1a is not supported by the spring, an optimum amount of stimulus can be given to the surface of the organism either automatically or manually, and the lancet 3 can invade the surface of the organism in optimum timing by the pressing pressure of the spring 2. Furthermore, it is not necessary to inject an injection solution from the lancet 3, and therefore the time period over which the lancet 3 invades the surface of the organism is very short compared to the injection needle of the injector.

As a result of the difference described above, for the injector disclosed in Japanese Patent Laid-Open No. 2001-161815, it is very difficult to obtain an effect of alleviating a pain compared to the invasive apparatus 10a of this embodiment.

Furthermore, the injector in Japanese Patent Laid-Open No. 2001-161815 has a disadvantage that a periphery of an injection area is pressed, resulting in difficulty to inject a drug, and therefore a function specific to the injector is impaired.

On the other hand, in this embodiment, a body fluid such as blood can easily be effused by opening puncturing means after puncture in the case of puncture. In this way, the invasive apparatus of this embodiment is fundamentally different from the injector in Japanese Patent Laid-Open No. 2001-161815.

Furthermore, in this embodiment, blood is collected by puncturing the surface of the organism with the lancet 3, but the present invention is not limited thereto, and an in vivo fluid other than blood may be collected. What is essential is collection of an invivo fluid by puncturing the surface of the organism with the lancet 3 irrespective of whether the in vivo fluid is blood or not.

In this way, the invasive apparatus 10a of this embodiment is provided with means of giving to the surface of the organism a mechanical stimulus different from a stimulus given by the lancet 3 as shown with the stimulus applying portion 1a, aside from means of puncturing the surface of the organism by the lancet 3 or the like to make an invasion, for obtaining an in vivo fluid with respect to the organism.

If an in vivo fluid is obtained with respect to the organism using the invasive apparatus 10a of this embodiment, a mechanical stimulus different from a stimulus given by means of puncturing the surface of the organism with the lancet 3 to make an invasion is given to the surface of the organism by the stimulus applying portion 1a, thus making it possible to inject a reagent or collect blood with a very small pain.

That is , the invasive apparatus 10a of this embodiment is provided with the stimulus applying portion 1a giving a stimulus to the surface of the organism aside from the lancet 3 using a needle and the like when the surface of the organism is punctured for obtaining an in vivo fluid with respect to the organism.

Use of a hollow tube as the lancet 3 of the invasive apparatus 10a makes it easy to obtain an in vivo fluid with respect to the organism. By using the hollow tube as the lancet 3, an in vivo fluid can be collected with an alleviated pain.

Furthermore, for indirectly obtaining an in vivo fluid, if the lancet 3 is a knife or needle, blood can be effused on the surface, and easily introduced into a diagnosis apparatus or the like. That is, this allows a very small amount of blood to be collected easily and withanalleviatedpain. Furthermore, it is also possible to use a laser beam instead of the lancet 3, and blood can be effused on the surface of the skin, and easily introduced into the diagnosis apparatus or the like.

This allows a mechanical stimulus to be given to the surface of the organism (skin) with a simple configuration to stimulate other cutaneous sense different from a pain, of cutaneous receptors, to mask a painful stimulus.

That is, the mechanical stimulus given to the surface of the organism by the stimulus applying portion 1a can activate the actions of Meissner's corpuscles and Pacinian corpuscles of the human body so that a pain according to the gate control theory explained in physiology is hard to be felt.

Furthermore, Meissner's corpuscles and Pacinian corpuscles that are cutaneous receptors exist at a depth of 3 mm or less from the surface of the organism, i.e. the surface of the skin. For a stimulus at a depth of 3 mm or less from the surface of the organism, i.e. the surface of the skin, the sense of touch and the sense of pain pass through the same neural transmission pathway to transmit the sense of the stimulus to the brain. Thus, a stimulus is given to the surface of the organism, i.e. the surface of the skin with the stimulus applying portion 1a or the like, and then the surface of the organism, i.e. the surface of the skin is invaded to a depth of 3 mm or less, whereby the sense of touch by the stimulus of the stimulus applying portion 1a is transmitted to the brain, but the sense of pain by the lancet 3 is prevented from being transmitted to the brain, and thus a pain can be harder to be felt.

In other words, the sense of touch is stimulated with a puncturing device such as the lancet 3 to feel a pain, but before or in synchronization with the processing, a different stimulus is given to sensory receptors of the sense of touch and the sense of pressure, whereby a gate control controlling pathway is activated at the posterior horn of spinal cord, so that a pain is hard to be felt in a pain transmission pathway.

The invasive apparatus 10a gives a stimulus different from a stimulus by the lancet 3 to the surface of the organism by the stimulus applying portion 1a for obtaining an in vivo fluid with respect to the organism.

The invasive apparatus 10a operates as described above, whereby a mechanical stimulus can be given the surface of the organism (skin) with a simple configuration to stimulus other cutaneous sense different from a pain, of cutaneous receptors, to mask a painful stimulus.

That is, the actions of Meissner's corpuscles and Pacinian corpuscles can be activated with the mechanical stimulus to the surface of the organism by the stimulus applying portion 1a, so that a pain according to the gate control theory explained in physiology is hard to be felt. In other words, the sense of pain is stimulated with a puncturing device such as a needle to feel a pain, but before or in synchronization with the processing, a different stimulus is given to sensory receptors of the sense of touch and the sense of pressure, whereby a gate control controlling pathway can be activated at the posterior horn of spinal cord, so that a pain is hard to be felt in a pain transmission pathway.

Furthermore, in this embodiment, a mechanical stimulus is given to the surface of the organism by the stimulus applying portion 1a, but the present invention is not limited thereto, and an electric stimulus, optical stimulus or stimulus by a change in temperature may be given.

Furthermore, a stimulus by the stimulus applying portion 1a is given from a time when or before the leading end of the lancet 3 or the like contacts the surface of the organism, whereby a pain is easily alleviated. Furthermore, because the stimulus applying portion 1a exists closer to the organism side than the leading end of the lancet 3 spatially, it becomes easy to give a stimulus in advance.

Furthermore, if the position at which a stimulus is given by the stimulus applying portion 1a is at a distance of at least 20 mm or less from an area in which the lancet 3 contacts the surface of the organism, a pain can be effectively alleviated. Particularly if a finger tip is punctured, Meissner's corpuscles and Pacinian corpuscles that are cutaneous receptors densely exist, and therefore if the position at which a stimulus is given by the stimulus applying portion 1a is at a distance of 1 mm to 10 mm from an area in which the lancet 3 contacts the surface of the organism, a pain can be effectively alleviated. Further, if the finger tip is punctured, it is more effective that the position at which a stimulus is given by the stimulus applying portion 1a is at a distance of 3 mm to 6 mm from an area in which the lancet 3 contacts the surface of the organism. Furthermore, this aspect may be applied to not only the first embodiment but also other embodiments in the same way as a matter of course.

In Figures 1 (b) and 1(c), invasive apparatuses different in configuration of the stimulus applying portion 1a from the invasive apparatus 10a of Figure 1 (a) are shown. Specifically, the invasive apparatus 10b of Figure 1(b) comprises a stimulus applying portion 1b instead of the stimulus applying portion 1a. For the stimulus applying portion 1b, the leading end portion thereof has a shape having irregularities in the orthogonal direction of the surface of the organism, i.e. a shape waving in the direction orthogonal to the surface of the organism.

Furthermore, the invasive apparatus 10c of Figure 1(c) comprises a stimulus applying portion 1c instead of the stimulus applying portion 1a. The stimulus applying portion 1c is configured to have discontinuous protrusions on the outer periphery of the center of the lancet passage port 7.

In this way, as shown with the stimulus applying portions 1a, 1b and 1c, the stimulus applying portion is desirably situated continuously or discontinuously on the outer periphery of an area in which the lancet 3 contacts the surface of the organism.

Furthermore, in contrast with what is described above, the lancet may be situated on the outer periphery of the stimulus applying portion.

As shown in the invasive apparatus 10c of Figure 1(c), it is important that the stimulus applying portion 1c exists at least one location.

Furthermore, it is desirable that the stimulus applying portions 1a, 1b and 1c shown in Figures 1(a), 1(b) and 1(c) each have a raised portion of 0.1 mm or greater, and the angle of contact with the surface of the organism is 5 degrees or greater. Further, it is most desirable that they each have a raised portion of 0.5 mm to 2 mm, and the angle of contact with the surface of the organism is 80 to 100 degrees. Furthermore, this aspect may be applied to not only the first embodiment but also other embodiments in the same way as a matter of course.

Furthermore, as described above, it is desirable that the stimulus applying portion 1a protrudes before the lancet 3 contacts the surface of the organism.

Furthermore, if the invasive apparatuses 10a, 10b and 10c of this embodiment, and the like are used as lancet apparatuses of collecting blood, a needle or knife may be used as the lancet 3, and a laser beam may be used instead of the lancet 3. In this way, if the invasive apparatuses 10a, 10b and 10c of this embodiment are used as lancet apparatuses of collectingblood, it is desirable that irregularities are provided in an outer periphery at a distance of at least 10 mm or less from an area in which the lancet 3 contacts the surface of the organism, and at least 1 kgf/cm² of pressure is applied as a mechanical stimulus in advance before and while blood is collected.

Moreover, it is desirable that the load application speed is 0.1 kgf/sec·cm² or greater, and the maximum load application point is 0.1 to 5 seconds before puncture-driving. Furthermore, this aspect may be applied to not only the first embodiment but also other embodiments in the same way as a matter of course.

Furthermore, as the lancet 3, single or multiple needles, hollow needles, needles with sawtooth wave-like sides, needle fine knives for acupuncture, etc. are illustrated. The length of the lancet 3 is preferably about 100 µm to several mm, but is not specifically limited. Furthermore, this aspect may be applied to not only the first embodiment but also other embodiments in the same way as a matter of course.

Furthermore, any location of the lancet 3 is sufficient as long as it is placed at the center of the stimulus applying portion, the peripheral edge portion thereof or the like, and a stimulus can be used to puncture the skin painlessly and effectively.

For the operation of releasingpuncture, for example, an operation of pulling out a needle, or the like is shown if the lancet 3 is a needle. There are various configurations of releasing puncture of the lancet 3, and they include a configuration in which the lancet 3 is manually released, and a configuration in which all or part of releasing of puncture of the lancet 3 and the like, and series of operations including a suction operation are automatically performed.

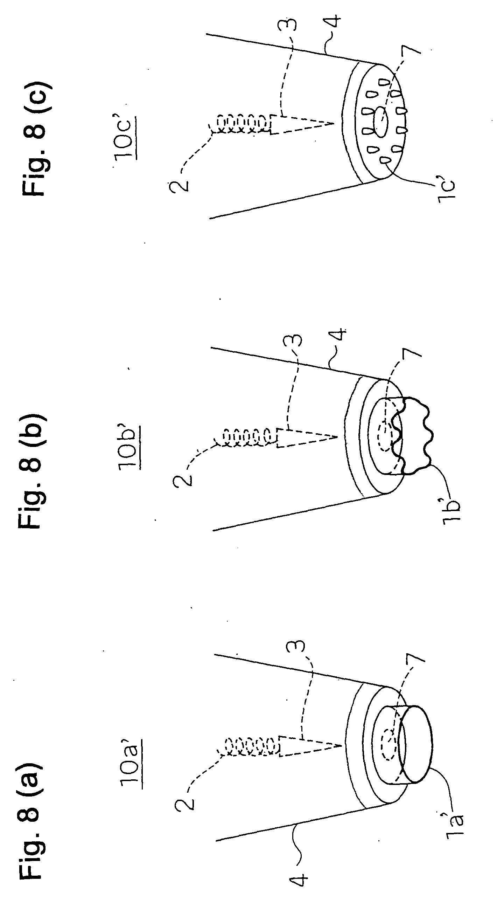

Furthermore, the stimulus applying portions 1a, 1b and 1c may be made each detachable, so that the stimulus applying portions 1a, 1b and 1c can be replaced with new stimulus applying portions 1a, 1b and 1c each time blood is collected, thereby making it possible to avoid a problem such that the stimulus applying portions 1a, 1b and 1c are contaminated with blood effused from the surface of the organism. Invasive apparatuses 10a', 10b' and 10c' having detachable stimulus applying portions 1a' , 1b and 1c' are shown in Figures 8(a), 8(b) and 8(c), respectively. For example, the stimulus applying portion 1a' of Figure 8(a) is detachable from the holder 4 at the boundary with the holder 4. Thus, if the stimulus applying portion 1a' is contaminated with blood, it can be easily replaced with the stimulus applying portion 1a' which is not contaminated with blood. The invasive apparatuses 10b' and 10C' can be treated in the same manner as the invasive apparatus 10a'. Furthermore, this aspect may be applied to not only the first embodiment but also stimulus applying portions for use in other embodiments as a matter of course.

### (Second Embodiment)

The second embodiment will now be described.

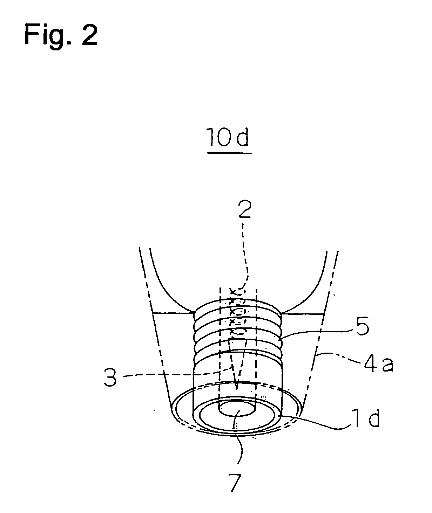

An invasive apparatus 10d of the second embodiment is shown in Figure 2. The invasive apparatus 10d is, for example, a lancet apparatus of collecting blood.

The invasive apparatus 10d is comprised of a stimulus applying portion 1d, a spring 2, a lancet 3, a holder 4a, a spring 5 and a lancet passage port 7.

Furthermore, same parts as those in the first embodiment are given same symbols and detailed descriptions thereof are not presented.

The stimulus applying portion 1d is means of giving to the surface of an organism a stimulus different from a stimulus given to the surface of the organism when the lancet 3 invades the surface of the organism, and the stimulus applying portion 1a is made of material such rubber, plastic or metal, and has a certain strength.

The holder 4a is an enclosure storing the lancet 3.

The spring 5 is a helical spring grounded at the rear of the stimulus applying portion 1d.

Specifically, the lancet passage port 7 is provided in the lower part of the holder 4a, and the stimulus applying portion 1d is provided in an outer periphery portion at a predetermined distance from the center of the lancet passage port 7. Furthermore, the spring 5 is placed in the upper part of the stimulus applying portion 1d, and the stimulus applying portion 1d is capable of being protruded by a pressing pressure of the spring 5. Furthermore, the lancet 3 is stored in the holder 4, and is capable of being protruded from the lancet passage port 7 by the spring 2.

The operations of this embodiment will now be described.

For collecting blood from the surface of the organism by the invasive apparatus 10d, the invasive apparatus 10d is first brought close to an area of blood collection on the surface of the organism.

Then, the stimulus applying portion 1d of the invasive apparatus 10d is pressed against the periphery of the area of blood collection on the surface of the organism. Consequently, the stimulus applying portion 1d gives a mechanical stimulus to the surface of the organism by a pressing pressure of the spring 5 before the lancet 3 is protruded. Furthermore, the amount of mechanical stimulus given to the surface of the organism by the stimulus applying portion 1d at this time can be adjusted by the spring 5, and is adjusted so that a pain felt when the lancet 3 punctures the surface of the organism to make an invasion is reduced to a minimum. That is, the amount of mechanical stimulus given to the surface of the organism by the stimulus applying portion 1d is adjusted to be at least 1 kgf/cm² as a pressure. In this way, by using the spring 5, no excessive pressure is applied to the skin.

After the expiration of a predetermined time interval from a time when the stimulus applying portion 1d gives a mechanical stimulus to the surface of the organism, the lancet 3 is protruded from the lancet passage port 7 by a pressing pressure of the spring 2 to puncture the surface of the organism to make an invasion. Consequently, blood is effused from the punctured surface of the organism. Then, the blood effused from the surface of the organism is collected.

In this way, unlike the first embodiment, the spring 5 is attached to the upper part of the stimulus applying portion 1d to adjust the amount of mechanical stimulus given by the stimulus applying portion 1d, whereby the amount of mechanical stimulus can be adjusted, thus making it possible to further reduce a pain.

Furthermore, in this embodiment, a helical spring is used as the spring 5, but the present invention is not limited thereto, and an elastic member such as rubber, electromagnetic force, vacuum suction force or the like may be used.

Furthermore, in this embodiment, when the stimulus applying portion 1d contacts the surface of the organism to give a mechanical stimulus to the surface of the organism, the stimulus applying portion 1d leaps up toward the upper part, i.e. the inner side of the holder 4a by a reactive force from the surface of the organism. Thus, it is also possible to detect that the stimulus applying portion 1d has leaped toward the upper part by the reactive force from the surface of the organism, and to cause the lancet 3 to protrude from the lancet passage port 7 to invade the surface of the organism in timing of the detection.

### (Third Embodiment)

The third embodiment will now be described.

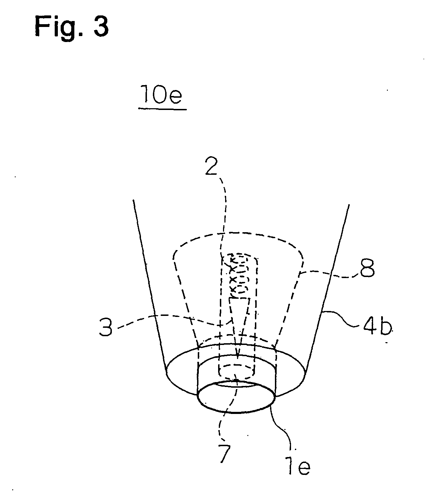

An invasive apparatus 10e of the third embodiment is shown in Figure 3. The invasive apparatus 10e is, for example, a lancet apparatus of collecting blood.

The invasive apparatus 10e is comprised of a stimulus applying portion 1e, a spring 2, a lancet 3, a holder 4b, a lancet passage port 7 and a vibration body 8.

Furthermore, same parts as those in the first embodiment are given same symbols and detailed descriptions thereof are not presented.

The stimulus applying portion 1e is means of giving to the surface of the organism a stimulus different from a stimulus given to the surface of the organism when the lancet 3 invades the surface of the organism, by vibration of the vibration body 8, and the stimulus applying portion 1e is made of material such as rubber, plastic or metal, and has a certain strength.

The holder 4b is an enclosure storing the lancet 3.

The vibration body 8 produces vibrations by a coil and an electromagnet, or produces vibrations by a piezoelectric element. The vibration body that is used is desirably provided with means of making the amplitude or frequency of the vibration body variable.

That is, the vibration body 8 is a vibration body capable of vibrating vertically and laterally the area of contact between the stimulus applying portion 1e and the surface of the organism. The vibration body 8 is configured to vibrate vertically and laterally in the lancet, and the vibration body 8 and the stimulus applying portion 1e closely contact each other.

It is also effective that the area of contact with the skin is provided with a material of high friction with the skin, such as rubber or plastic, or has a slightly protrusive form in order to prevent the shift of the skin.

Specifically, the lancet passage port 7 is provided in the lower part of the holder 4b, and the stimulus applying portion 1e is provided in an outer periphery portion at a predetermined distance from the center of the lancet passage port 7. Furthermore, the vibration body 8 is placed in the upper part of the stimulus applying portion 1e, so that the stimulus applying portion 1e can be vibrated. Furthermore, the lancet 3 is stored in the holder 4b, and is capable of being protruded from the lancet passage port 7 by the spring.

The operations of this embodiment will now be described.

For collecting blood from the surface of the organism by the invasive apparatus 10e, the invasive apparatus 10e is first brought close to an area of blood collection on the surface of the organism.

Then, the stimulus applying portion 1e of the invasive apparatus 10e is pressed against the periphery of the area of blood collection on the surface of the organism, and the vibration body 8 is vibrated. Consequently, the stimulus applying portion 10d is vibrated by the vibration body 8, and therefore gives a mechanical discontinuous stimulus to the surface of the organism. Furthermore, the amount of mechanical stimulus given to the surface of the organism by the stimulus applying portion 1e at this time and the cycle of vibrations can be adjusted by the vibration body 8, and is adjusted so that a pain felt when the lancet 3 punctures the surface of the organism to make an invasion is reduced to a minimum. That is, the amount of mechanical stimulus given to the surface of the organism by the stimulus applying portion 1e is adjusted to be at least 1 kgf/cm² as a pressure.

After the expiration of a predetermined time interval from a time when the stimulus applying portion 1e gives a mechanical stimulus to the surface of the organism, the lancet 3 is protruded from the lancet passage port 7 by a pressing pressure of the spring 2 to puncture the surface of the organism to make an invasion. Consequently, blood is effused from the punctured surface of the organism. Then, the blood effused from the surface of the organism is collected.

In this way, unlike the first embodiment, the vibration body 8 is attached to the upper part of the stimulus applying portion 1e to adjust the amount of mechanical stimulus given by the stimulus applying portion 1e, whereby the amount of mechanical stimulus given to the surface of the organism can be adjusted, thus making it possible to further reduce a pain.

Furthermore, the amplitude or frequency of the vibration body that is used as the vibration body 8 is made variable, whereby the amplitude of frequency can be adjusted so that a pain by the lancet 3 can be further reduced.

The invasive apparatuses of the first and second embodiments continuously give a stimulus different from a stimulus by the lancet 3 to the surface of the organism. In contrast to this, the invasive apparatus 10e of the third embodiment enables blood collection to be performed by intermittently giving a stimulus different from a stimulus by the lancet 3 to a local spot of the surface of the skin of the organism by vibration of the vibration body to invade the surface of the organism with the lancet 3, thus making it possible to easily alleviate a pain significantly.

That is, if a stimulus different from a stimulus by the lancet 3 is continuously given for a long time, the effect of alleviating a pain from invasion with the lancet 3 is lost. However, by changing a stimulus different from a stimulus by the lancet 3 using the vibration body as in this embodiment, the effect of alleviating a pain from invasion by the lancet 3 can be obtained even if an invasion is made with the lancet 3 in any timing.

In this way, a mechanical stimulus by vibrations is given when the mechanical stimulus is given to the surface of the organism by vibrating the stimulus applying portion 1e by the vibration body 8, whereby the surface of the organism given the stimulus is never rubbed, and thus the surface of the organism is never damaged.

Furthermore, by providing means of keeping almost constant a stimulus to the surface of the organism to eliminate a difference in angle of application of the apparatus to the surface of the organism and a difference in pressing pressure from person to person and by irregularities of the surface of the organism, the following problem can be avoided. That is, the problem is that a stimulus to the surface of the organism by the vibration body is reduced and the masking effect is varied to increase a pain, or a stimulus to the surface of the organism increases to worsen the touch of the surface of the organism.

Furthermore, if the vibration body 8 produces vibrations by a coil and an electromagnet, or produces vibrations by a piezoelectric element, a mechanical stimulus can be given to the skin by vibrations with a simple configuration.

Furthermore, the amplitude or frequency of vibrations of the vibration body 8 is made variable by providing means of making variable the amplitude or frequency of the vibration body 8, whereby vibrations of the vibration body 8 can be adjusted in accordance to a user to obtain vibrations appropriate to the user.

Furthermore, the pressure applied to the surface of the organism by the vibration body 8 is desirably at least 1 kgf/cm². This allows a painful stimulus to be masked effectively.

### (Fourth Embodiment)

The fourth embodiment will now be described.

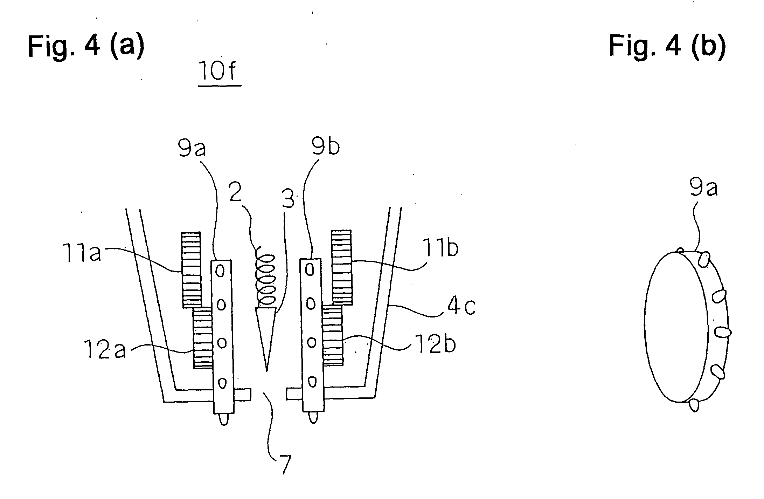

An invasive apparatus 10f of the fourth embodiment is shown in Figure 4. The invasive apparatus 10f is, for example, a lancet apparatus of collecting blood.

In Figure 4(a), the invasive apparatus 10 f is comprised of stimulus applyingportions 9a and 9b, a spring 2, a lancet 3, a holder 4c, a lancet passage port 7 and gears 11a, 12a, 11b and 12b.

Furthermore, same parts as those in the first embodiment are given same symbols and detailed descriptions thereof are not presented.

The stimulus applying portions 9a and 9b are rolling bodies each giving a stimulus, which is different from a stimulus given to the surface of the organism when the lancet 3 invades the surface of the organism, to the surface of the organism by rolling, are made of material such as rubber, plastic or metal, and have a certain strength. Figure 4 (b) is a perspective view of the stimulus applying portion 9a. It can be understood that protrusions are formed on an outer periphery portion of the stimulus applying portion 9a, and the protrusions can give a stimulus different from a stimulus by the lancet 3 to the surface of the organism as the stimulus applying portion 9a rolls. The stimulus applying portion 9b has a configuration same as that of the stimulus applying portion 9a.

That is, the stimulus applying portions 9a and 9b can give a stimulus to the surface of the organism by rolling because of irregularities on the surface. The irregularities have a diameter of 1 mm and a height of 1 mm, and are at a distance of 3 mm from the punctured area of the skin on both sides. A material of high friction with the skin, such as rubber or plastic, may be placed on an area of contact with the skin.

The holder 4c is an enclosure storing the lancet 3.

The gears 11a and 12a are means of rolling the stimulus applying portion 9a.

The gears 11b and 12b are means of rolling the stimulus applying portion 9b.

Specifically, the lancet passage port 7 is provided in the lower part of the holder 4c, and the stimulus applying portions 9a and 9b are provided in an outer periphery portion at a predetermined distance from the center of the lancet passage port 7. Furthermore, the stimulus applying portion 9a is in a state in which it can roll by transmission of a power thereto by the gears 11a and 12a. Similarly, the stimulus applying portion 9b is in a state in which it can roll by transmission of a power thereto by the gears 11b and 12b. Furthermore, the lancet 3 is stored in the holder 4b, and is capable of being protruded from the lancet passage port 7 by the spring 2.

The operations of this embodiment will now be described.

For collecting blood from the surface of the organism by the invasive apparatus 10f, the invasive apparatus 10f is first brought close to an area of blood collection on the surface of the organism.

Then, the stimulus applying portions 9a and 9b of the invasive apparatus 10f are pressed against the periphery of the area of blood collection on the surface of the organism, and the stimulus applying portions 9a and 9b roll by a power transmitted from the gears 11a and 12a and gears 11b and 12b.

In this way, the stimulus applying portions 9a and 9b roll, and thus a discontinuous mechanical stimulus is given to the surface of the organism. Furthermore, the amount of mechanical stimulus given to the surface of the organism by the stimulus applying portions 9a and 9b and the cycle of vibrations can be adjusted by adjusting the rotation speed at which the stimulus applying portions 9a and 9b roll, and can be adjusted so that a pain felt when the lancet 3 punctures the surface of the organism to make an invasion is reduced to a minimum. That is, the amount of mechanical stimulus given to the surface of the organism by the stimulus applying portions 9a and 9b is adjusted to be at least 1 kgf/cm² as a pressure.

After the expiration of a predetermined time interval from a time when the stimulus applying portions 9a and 9b give a mechanical stimulus to the surface of the organism, the lancet 3 is protruded from the lancet passage port 7 by a pressing pressure of the spring 2 to puncture the surface of the organism to make an invasion. Consequently, blood is effused from the punctured surface of the organism. Then, the blood effused from the surface of the organism is collected.

In this way, unlike the first embodiment, rolling bodies are used as the stimulus applying portions 9a and 9b, whereby a discontinuous stimulus can be given to the surface of the organism. This stimulus can further reduce a pain by the lancet 3.

That is, if a stimulus different from a stimulus by the lancet 3 is continuously given for a long time, the effect of alleviating a pain from invasion with the lancet 3 is lost. However, by changing a stimulus different from a stimulus by the lancet 3 using the rolling body as in this embodiment, the effect of alleviating a pain from invasion by the lancet can be obtained even if an invasion is made with the lancet 3 in any timing.

In this way, in this embodiment, the stimulus applying portions 9a and 9b are constituted by rolling bodies rolling on the surface of the organism, but a rolling body with the center of gravity of the rolling body decentered with respect to the center of rotation of the rollingbody can be used as the rolling body. Furthermore , the pressure applied to the surface of the organism by the mechanical stimulus with the rollingbody is desirably at least 1 kgf/cm².

That is, the stimulus applying portions 9a and 9b are constituted by rolling bodies rolling on the surface of the organism, and therefore the rolling body is rolled on the surface of the organism to give a mechanical stimulus, whereby the surface of the organism given the stimulus is never rubbed, and thus the surface of the organism is never damaged.

Furthermore, the center of gravity of the rolling body as the stimulus applying portions 9a and 9b is desirably decentered with respect to the center of rotation of the rolling body. By providing such a configuration, the mechanical stimulus to the surface of the organism can be changed.

### (Fifth Embodiment)

The fifth embodiment will now be described.

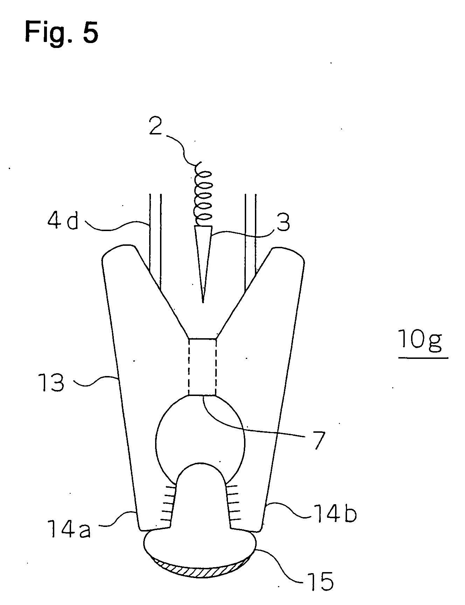

An invasive apparatus 10g of the fifth embodiment is shown in Figure 5.

The invasive apparatus 10 g is comprised of a spring 2, a lancet 3, a holder 4d, a stimulus applying portion 13 and a lancet passage port 7. The stimulus applying portion 13 has leading end portions 14a and 14b at its end.

The invasion 10 g is, for example, a lancet apparatus of collecting blood.

Furthermore, same parts as those in the first embodiment are given same symbols and detailed descriptions thereof are not presented.

The stimulus applying portion 13 is like a type of binder for giving to a finger 15 a stimulus different from a stimulus given to the surface of the organism when the lancet 3 invades the surface of the organism by sandwiching the finger 15 by the leading end portions 14a and 14b, and the stimulus applying portion 13 is made of material such as rubber, plastic or metal, and has a certain strength.

The holder 4d is an enclosure storing the lancet 3.

Specifically, the lancet passage port 7 is provided in the stimulus applying portion 13 as shown in Figure 5 , and the leading end portions 14a and 14b of the stimulus applying portion 13 are provided in an outer periphery portion at a predetermined distance from the center of the lancet passage port 7. Furthermore, the lancet 3 is stored in the holder 4d, and is capable of being protruded from the lancet passage port 7 by the spring 2.

The operations of this embodiment will now be described.

For collecting blood from the surface of the organism by the invasive apparatus 10g, the invasive apparatus 10g is first brought close to an area of blood collection in the finger 15.

Then, the finger 15 is sandwiched by the leading end portions 14a and 14b of the stimulus applying portion 13 of the invasive apparatus 10g. Consequently, a stimulus different from a stimulus given to the finger 15 by the lancet 3 can be given to the finger 15. Furthermore, the amount of mechanical stimulus given to the finger by the stimulus applying portion 13 is adjusted to be at least 1 kgf/cm² as a pressure.

After the expiration of a predetermined time interval from a time when the stimulus applying portion 13 gives a mechanical stimulus to the finger 15, the lancet 3 is protruded from the lancet passage port 7 by a pressing pressure of the spring 2 to puncture the finger 15 to make an invasion. Consequently, blood is effused from the punctured finger 15. Then, the blood effused from the finger 15 is collected.

In this way, unlike the first embodiment, a type of binder sandwiching the finger 15 is used as the stimulus applying portion 13, whereby a mechanical stimulus can be given to the surface of the organism. This stimulus can further reduce a pain by the lancet 3.

### (Sixth Embodiment)

The sixth embodiment will now be described.

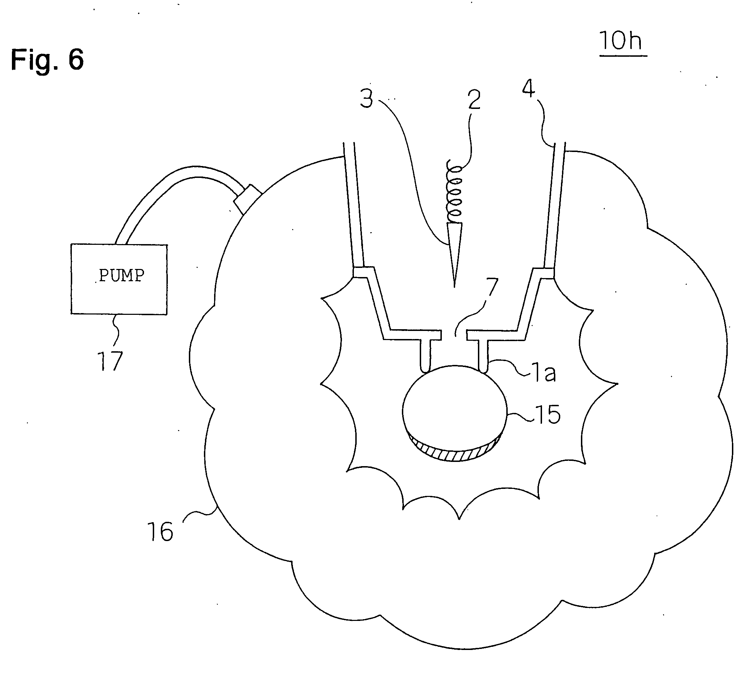

An invasive apparatus 10h of the sixth embodiment is shown in Figure 6.

The invasive apparatus 10h is comprised of a spring 2, a lancet 3, a holder 4, a stimulus applying portion 1a, a lancet passage port 7, a sealed air body 16 and a pump 17.

The invasive apparatus 10g is, for example, a lancet apparatus of collecting blood.

Furthermore, same parts as those in the first embodiment are given same symbols and detailed descriptions thereof are not presented.

The sealed air body 16 is means of oppressing a finger 15 with air encapsulated from the pump 17 to press the finger 15 against the stimulus applying portion 1a. The sealed air body 16 is, for example, a cuff that can bring the stimulus applying portion 1a and the finger 15 into close contact with each other.

The pump 17 is means of encapsulating air in the sealed air body 16.

Specifically, the lancet passage port 7 is provided in the lower part of the holder 4, and the stimulus applying portion 1a is provided in an outer periphery portion at a predetermined distance from the center of the lancet passage port 7. Furthermore, the lancet 3 is stored in the holder 4, and is capable of being protruded from the lancet passage port 7 by the spring 2. The sealed air body 16 is connected to the pump 17, and the sealed air body 16 is capable of oppressing the finger 15. Furthermore, the stimulus applying portion 1a is provided in an outer periphery portion at a distance of 10 mm or less from the center of the lancet passage port 7.

The operations of this embodiment will now be described.

For collecting blood from the surface of the organism by the invasive apparatus 10h, the invasive apparatus 10h is first brought close to an area of blood collection on the surface of the organism.

Then, the stimulus applyingportion 1a of the invasive apparatus 10h is pressed against the periphery of the area of blood collection on the surface of the organism. Further, air is sent into the sealed air body 16 from the pump 17. Consequently, the finger 15 is oppressed by the sealed air body 16, and pressed against the stimulus applying portion 1a. In this way, the stimulus applying portion 1a gives a mechanical stimulus to the surface of the organism.

That is, air is previously sent into the sealed air body 16 by the pump 17 to expand the sealed air body 16, whereby the surface of the organism and the stimulus applying portion 1a are brought into close contact with each other, and a stimulus is given to alleviate a pain during blood collection. The area of contact with the skin may be provided with a material of high friction with the skin, such as rubber or plastic, or have a slightly protrusive form in order to prevent the shift of the skin.

After the expiration of a predetermined time interval from a time when the stimulus applying portion 1a gives a mechanical stimulus to the surface of the organism, the lancet 3 is protruded from the lancet passage port 7 by a pressing pressure of the spring 2 to puncture the surface of the organism to make an invasion. Consequently, blood is effused from the punctured surface of the organism. Then, the blood effused from the surface of the organism is collected.

In this way, in this embodiment, the finger 15 is oppressed with the sealed air body 16 from a time when or before the lancet 3 contacts the surface of the organism, whereby the finger 15 is pressed against the stimulus applying portion 1a to give a stimulus different from a stimulus by the lancet 3 by the stimulus applying portion 1a. This enables a painful stimulus to be masked in an early stage.

In this way, in the lancet apparatus of collecting blood, if irregularities are provided in an outer periphery at a distance of at least 10 mm or less from an area in which a needle or knife contacts the surface of the organism, a mechanical stimulus is given to a lancing portion by the sealed air body such as a cuff in advance before and while blood is collected, blood can be collected with a pain significantly alleviated.

Furthermore, by providing means of keeping almost constant a stimulus to the surface of the organism to eliminate a difference in angle of application of the apparatus to the surface of the organism, a difference in pressing pressure from person to person and difference in irregularities of the surface of the organism, the following problem can be avoided. That is, the problem is that a stimulus to the surface of the organism by the stimulus applying portion 1a is reduced and the masking effect is varied to increase a pain, or a stimulus to the surface of the organism increases to worsen the touch of the surface of the organism.

Furthermore, the invasive apparatus 10 h of this embodiment comprises the stimulus applying portion 1a in the above description, but it may comprise no stimulus applying portion 1a. Even such a configuration enables a pain by the lancet 3 to be alleviated because the sealed air body 16 oppresses the finger 15 to give a stimulus different from a stimulus by the lancet 3.

### (Seventh Embodiment)

The seventh embodiment will now be described.

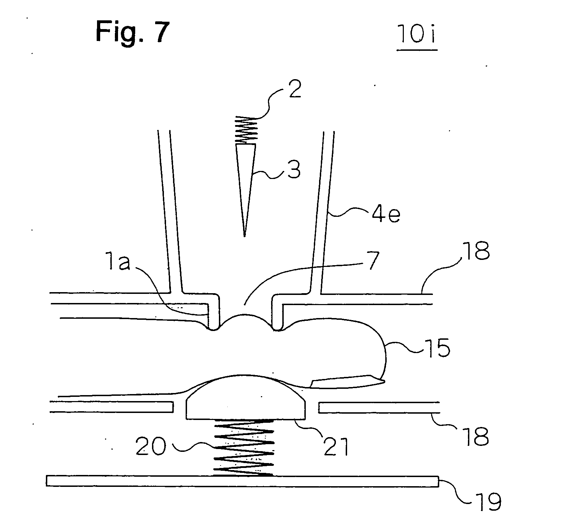

An invasive apparatus 10i of the seventh embodiment is shown in Figure 7.

The invasive apparatus 10i is comprised of a spring 2, a lancet 3, a holder 4, a stimulus applying portion 1a, a lancet passage port 7, a finger insertion guiding means 20, a finger holding means 21, a spring 20 and a bottom plate 19.

The invasive apparatus 10i is, for example, a lancet apparatus of collecting blood.

Furthermore, same parts as those in the first embodiment are given same symbols and detailed descriptions thereof are not presented.

The holder 4e is an enclosure storing the lancet 3.

The finger insertion guiding means 18 is means of inserting a finger 15 to collect blood.

The finger holding means 21 is means of pressing the finger 15 against the stimulus applying portion 1a by pressing the finger 15 on the side opposite to an area of collection of blood.

The spring 20 is means of pressing the finger holding means 21 against the side of the finger 15.

Specifically, the lancet passage port 7 is provided in the lower part of the holder 4e, and the stimulus applying portion 1a is provided in an outer periphery portion at a predetermined distance from the center of the lancet passage port 7. Furthermore, the lancet 3 is stored in the holder 4, and is capable of being protruded from the lancet passage port 7 by the spring 2. Furthermore, the finger 15 to collect blood is capable of being inserted into the finger insertion guiding means 18, and the finger holding means 21 is provided on the side opposite to the stimulus applying portion 1a of the finger insertion guiding means 18. The finger holding means 21 is means of pressing the finger 15, provided with a pressing pressure by the spring 20 which is attached to the bottom plate 19, against the side of the stimulus applying portion 1a.

The operations of this embodiment will now be described.

For collectingblood from the finger 15 by the invasive apparatus 10i, the finger 15 is first inserted into the finger insertion guiding means 18 of the invasive apparatus 10i.

Then, the finger 15 on the side opposite to an area in which blood is collected is pressed against the side of the stimulus applyingportion 1a with the finger holding means 21. Consequently, the stimulus applying portion 1a gives a mechanical stimulus to the surface of the finger 15.

After the expiration of a predetermined time interval from a time when the stimulus applying portion 1a gives a mechanical stimulus to the finger 15, the lancet 3 is protruded from the lancet passage port 7 by a pressing pressure of the spring 2 to puncture the surface of the organism to make an invasion. Then, blood is collected through the lancet 3 from the surface of the finger 15.

In this way, in this embodiment, a stimulus different from a stimulus by the lancet 3 is given by the stimulus applying portion 1a from a time when or before the lancet 3 contacts the surface of the finger 15. This enables a painful stimulus to be masked in an early stage.

In this way, the invasive apparatus 10i of this embodiment has the stimulus applying portion 1a, the finger insertion guiding means 18 and the finger holding means 21, is provided with the stimulus applying portion 1a on an outer periphery at a distance of at least 10 mm or less from an area in which a needle, knife or the like contacts the surface of the organism, and holds down the finger from back with a spring or air for giving a mechanical stimulus in advance before blood is collected, whereby blood can be collected with a pain significantly alleviated.

Furthermore, by providing means of keeping almost constant a stimulus to the surface of the organism to eliminate a difference in angle of application of the apparatus to the surface of the organism, a difference in pressing pressure from person to person and a difference in irregularities of the surface of the organism, the following problem can be avoided. That is, the problem is that a stimulus to the surface of the organism by the stimulus applying portion 1a is reduced and the masking effect is varied to increase a pain, or a stimulus to the surface of the organism increases to worsen the touch of the surface of the organism.

Further, the finger holding means 21 may be vibrated. That is, the following effect can be obtained by vibrating the finger holding means 21. That is, if a stimulus different from a stimulus by the lancet 3 is continuously given for a long time, the effect of alleviating a pain from invasion with the lancet 3 is lost. However, a stimulus different from a stimulus by the lancet 3 can be changed by vibrating the finger holding means 21 as in this embodiment, thus making it possible to obtain the effect of alleviating a pain from invasion by the lancet even if an invasion is made with the lancet 3 in any timing.

Further, the organism insertion guiding means of the present invention is not limited to the finger insertion guiding means 18 in this embodiment, but may be means of inserting an arm or means of inserting a leg. In short, the organism insertion guiding means of the present invention may be any means as long as it is intended for inserting an organism.

Further, the organism of the present invention is not limited to the finger 15 in this embodiment, but may be any part of an organism such as an arm, leg or abdomen.

Furthermore, the finger insertion guiding means 18 of this embodiment is an example of organism insertion guiding means of the present invention, and the finger holding means 21 of this embodiment is an example of organism holding means of the present invention.

Furthermore, in this embodiment, the stimulus applying portion gives a mechanical stimulus to the surface of the organism, but the present invention is not limited thereto, and the stimulus applying portion maybe anymeans of stimulating the surface of the organism, and examples thereof include, but is not specifically limited to, means of manually or automatically performing pressing functions optically or electrically.

Furthermore, the present invention can provide an apparatus measuring an measurement object in blood comprising a blood collecting unit to collect blood, stimulus giving means and a measurement unit, wherein irregularities are provided in an outer periphery at a distance of at least 10 mm or less from an area in which a needle, knife or the like contacts the surface of the organism, a mechanical stimulus is given in advance before blood collection and then blood is collected, the blood is introduced into a measurement unit by a hollow needle, and then a measurement object is measured in the measurement unit. As a result, a blood examination apparatus can be easily provided with a pain alleviated.

As described above, according to this embodiment, blood can easily be effused on the skin by the stimulus applying portion to secure an amount of blood required for diagnosis. Blood can be collected while giving a stimulus by the stimulus applying portion, thus making it possible to collect blood painlessly.

Further, the motion of the leading end of a needle for collection of blood can be prevented from being seen from a person subjected to blood collection when blood is collected, thus making it possible to collect blood without causing a sensation of fear or anxiety felt when the needle for collection of blood is inserted.

In this way, according to this embodiment, an injector or lancet of significantly alleviated pain can be obtained.

Furthermore, an in vivo fluid measuring apparatus comprising the invasive apparatus of the present invention, fluid transporting means of transporting an in vivo fluid obtained by invasion, and a measurement unit, wherein the measurement unit analyses the collected in vivo fluid, also belongs to the present invention.

Furthermore, the fluid transporting means includes, for example, means of performing passive transportation using capillarity, and means of performing active transportation using a pump or the like.

### Industrial Applicability

As apparent from what has been described above, the present invention can provide an invasive apparatus causing less pain, an invasive method and an in vivo fluid measuring apparatus.

Furthermore, the present invention can provide an in vivo fluid measuring apparatus that can measure an object in blood more accurately.

## Claims

1. An invasive apparatus of obtaining an in vivo fluid, comprising a different stimulus applying portion of applying a stimulus different from a stimulus applied by an invasive portion to an organism in a leading end area other than a hole through which said invasive portion goes out.

2. The invasive apparatus according to claim 1, wherein said different stimulus is a mechanical stimulus, electric stimulus, optical stimulus or change in temperature.

3. The invasive apparatus according to claim 2, wherein said different stimulus is a mechanical stimulus,
a recessed portion and/or a raised portion is formed in the area other than said hole,
said different stimulus applying portion is said recessed portion and/or raised portion, and
said different stimulus is given by the recessed portion and/or raised portion.

4. The invasive apparatus according to claim 3, wherein said recessed portion and/or raised portion is at a distance of from at least 1 mm to 20 mm from an area in which said invasive portion contacts the surface of the organism.

5. The invasive apparatus according to claim 4, wherein said surface of the organism is a finger tip, and
said recessed portion and/or raised portion is at a distance of from at least 1 mm to 10 mm from an area in which said invasive portion contacts said finger tip.

6. The invasive apparatus according to claim 5, wherein the invasive apparatus is a lancet apparatus of collecting blood.
